# EUROPÄISCHE PATENTSCHRIFT

(11) **EP 3 598 893 B1**
(45) Veröffentlichungstag und Bekanntmachung des Hinweises auf die Patenterteilung: **24.04.2024**
(21) Anmeldenummer: 19185294.6
(22) Anmeldetag: 09.07.2019
(51) Int. Cl.: A01K 5/02

(54) **VOLUMENDOSIERER, FÜTTERUNGSANLAGE UND VERFAHREN ZUR BEREITSTELLUNG VON FUTTER**
VOLUME METERING DEVICE, FEED SYSTEM AND METHOD FOR PROVIDING FEED
DOSEUR VOLUMÉTRIQUE, INSTALLATION D'ALIMENTATION POUR ANIMAUX ET PROCÉDÉ DE FOURNITURE DE L'ALIMENT POUR ANIMAUX

(30) Priorität: 26.07.2018 LU 100883
(43) Veröffentlichungstag der Anmeldung: 29.01.2020
(73) Patentinhaber: Big Dutchman International GmbH, 49377 Vechta (DE)
(72) Erfinder: Rolfes, Raphael, 26219 Bösel (DE)
(74) Vertreter: Eisenführ Speiser

(56) Entgegenhaltungen:
- CA-A1- 2 951 758
- DE-A1- 3 032 029
- GB-A- 1 323 636
- US-A- 742 969
- US-A- 3 144 173

## Beschreibung

Die Erfindung betrifft einen Volumendosierer für eine Fütterungsanlage zur Bereitstellung von Futter, insbesondere Trockenfutter für Schweine, insbesondere für die Einzeltierfütterung und eine solche Fütterungsanlage. Die Erfindung betrifft ferner ein variables Volumendosiersystem für eine Fütterungsanlage zur Bereitstellung von Futter, insbesondere Trockenfutter für Schweine, insbesondere für die Einzeltierfütterung. Die Erfindung betrifft ferner ein Verfahren zur Bereitstellung von Futter, insbesondere Trockenfutter für Schweine, insbesondere für die Einzeltierfütterung, sowie ein Verfahren zum Variieren eines Volumendosiersystems.

In der Nutztierhaltung, insbesondere in der Schweinehaltung, werden Fütterungsanlagen zur Bereitstellung von Futter für die Nutztiere eingesetzt. Insbesondere Fütterungsanlagen zur Bereitstellung von Trockenfutter sind bekannt. Beispielsweise wird bei der Sauenhaltung die restriktive Fütterung eingesetzt, die erfordert, dass dosierte Mengen an Futter dem einzelnen Nutztier zur Verfügung gestellt werden. Als Einzeltierfütterung wird insbesondere eine Fütterung bezeichnet, bei der einem einzelnen Nutztier eine dosierte Menge an Futter zur Verfügung gestellt wird, beispielsweise in einem Futtertrog, der nur für ein einzelnes Nutztier zugänglich ist.

Fütterungsanlagen umfassen in der Regel eine Futterzuführleitung zu Bereitstellung von Futter an eine Vielzahl von Futtertrögen, wobei das Futter in der Regel über ein Fallrohr in den Futtertrog gelangt. Die Bereitstellung von Futter über die Futterzuführleitung erfolgt in der Regel über ein Futterfördersystem und einen zugehörigen Antrieb. Beispielsweise weist die Futterzuführleitung eine in einem Förderrohr angeordnete, angetriebene Förderkette mit beabstandeten Mitnehmerscheiben auf. Diese Förderkette ist angetrieben und fördert Futter aus einem Futtervorrat entlang der Futterzuführleitung. Die Bereitstellung von Futter von der Futterzuführleitung in einen Futtertrog, gegebenenfalls über ein Fallrohr, erfolgt hingegen vorzugsweise gravitätisch. Unter einer gravitätischen Futterabgabe wird insbesondere eine Futterabgabe mittels Schwerkraft verstanden. Insbesondere erfolgt eine Futterabgabe von der Futterzuführleitung aus ohne einen weiteren Antrieb, sondern alleine durch Schwerkraft.

Zur Dosierung der an ein Nutztier bereitzustellenden Menge an Futter, beispielsweise für eine tagesrationierte Fütterung, werden in der Regel Volumendosierer eingesetzt. Diese werden über die Futterzuführleitung mit Futter beschickt. Am Volumendosierer kann in der Regel ein gewünschtes Volumen an Futter eingestellt werden. Nach dem Befüllen des am Volumendosierer eingestellten Volumens mit Futter aus der Futterzuführleitung kann das Futter dann, in der Regel gravitätisch, beispielsweise über ein Fallrohr, in einem Futtertrog für ein Nutztier bereitgestellt werden. Das Dokument CA2951758 A1 beschreibt so einen Volumendosierer.

Eine genaue Dosierung der den Nutztieren bereitgestellten Futtermengen ist für das Tierwohl und eine erfolgreiche Nutztierhaltung von großer Bedeutung. Gerade in bestimmten Phasen der Sauenhaltung ist oft die Bereitstellung einer sehr kleinen Futtermenge wünschenswert. Existierende Volumendosierer sind jedoch gerade für die Bereitstellung von sehr kleinen Futtermengen meist nicht geeignet, da eine solche Bereitstellung von Minimaldosierungen nicht oder nur unzuverlässig möglich ist.

Es ist daher eine Aufgabe der vorliegenden Erfindung, einen Volumendosierer für eine Fütterungsanlage zu Bereitstellung von Futter bereitzustellen, der einen oder mehrere der genannten Nachteile verringert oder beseitigt und/oder gegenüber existierenden Lösungen verbessert ist. Insbesondere ist es eine Aufgabe der vorliegenden Erfindung, einen Volumendosierer für eine Fütterungsanlage zur Bereitstellung von Futter bereitzustellen, der eine zuverlässige und/oder kostengünstige und/oder robuste Bereitstellung von Futter, insbesondere auch von sehr kleinen Futtermengen, ermöglicht. Ebenso ist es eine Aufgabe der vorliegenden Erfindung, eine Fütterungsanlage zu Bereitstellung von Futter, ein variables Volumendosiersystem für eine Fütterungsanlage zu Bereitstellung von Futter, ein Verfahren zu Bereitstellung von Futter und ein Verfahren zum Variieren eines Volumendosiersystems bereitzustellen, die ebenso gegenüber existierenden Lösungen verbessert sind.

Diese Aufgabe wird gelöst durch einen Volumendosierer nach Anspruch 1 für eine Fütterungsanlage zur Bereitstellung von Futter, insbesondere Trockenfutter für Schweine, insbesondere für die Einzeltierfütterung. Der Volumendosierer umfasst einen Futterbehälter mit einem ersten Behälterabschnitt mit einem Futtereingang und einem zweiten Behälterabschnitt mit einem verschließbaren Futterausgang zur gravitätischen Abgabe von Futter, wobei der erste Behälterabschnitt eine verschließbare Behälteröffnung aufweist, welche den ersten Behälterabschnitt mit dem zweiten Behälterabschnitt verbindet für einen gravitätischen Durchtritt von Futter aus dem ersten Behälterabschnitt in den zweiten Behälterabschnitt, wobei zwischen dem ersten und dem zweiten Behälterabschnitt eine höhenverstellbare Trennwand angeordnet ist, und wobei an der Trennwand ein Verschlusselement zum Verschließen der Behälteröffnung angeordnet ist.

Der Volumendosierer wird vorzugsweise in einer Fütterungsanlage zur Bereitstellung von Futter, insbesondere Trockenfutter für Schweine, insbesondere für die Einzeltierfütterung eingesetzt. Als Trockenfutter kommt vorzugsweise pelletiertes und/oder granulares und/oder krümeliges und/oder mehlförmiges Futter zum Einsatz. Der Volumendosierer dient insbesondere dazu, ein bestimmtes Volumen an Futter bereitzustellen. Hierzu ist es bevorzugt, dass bei einem Futterwechsel das Schüttgewicht, das auch als Litergewicht bezeichnet werden kann, des neuen Futters neu bestimmt wird, da unterschiedliche Futterarten und selbst verschiedene Chargen gleicher Futterarten ein unterschiedliches Schüttgewicht aufweisen können. Aus dem spezifischen Gewicht des einzusetzenden Futters und dem Futteranspruch der Nutztiere ergibt sich das zu verfütternde Futtervolumen. Eine Futtermenge wird hierin insbesondere als Futtervolumen verstanden.

Das Beschicken des Volumendosierers mit Futter kann vorzugsweise wie im Folgenden beschrieben erfolgen: Der Futterausgang wird verschlossen und der Futtereingang wird geöffnet. Über die Futterzuführleitung gelangt durch den Futtereingang nun Futter in den ersten Behälterabschnitt und gegebenenfalls auch in den zweiten Behälterabschnitt. Details zur Einstellung des Futtervolumens des Volumendosierers sind weiter unten beschrieben. Im Volumendosierers befindet sich nun eine auszudosierende Futtermenge. Durch Öffnen des Futterausgangs wird der Futterausgang freigegeben und das Futter im Volumendosierer wird gravitätisch aus dem Futterausgang abgegeben.

Der Volumendosierer umfasst einen Futterbehälter mit einem ersten und einem zweiten Behälterabschnitt. Der erste Behälterabschnitt weist einen Futtereingang auf, der insbesondere zur Übergabe von Futter aus einer Futterzuführleitung in den Futterbehälter ausgebildet ist. Auf diese Weise kann der Futterbehälter, insbesondere der erste Behälterabschnitt, über den Futtereingang mit Futter aus der Futterzuführleitung beschickt werden. Der zweite Behälterabschnitt weist einen Futterausgang auf, durch den Futter gravitätisch abgegeben werden kann, beispielsweise an ein Fallrohr und anschließend an einen Futtertrog. Der Futterausgang ist verschließbar, beispielsweise durch ein über einen Seilzug betätigte Gewichtselement, beispielsweise eine Verschlusskugel oder ein Verschlusskegel.

Der erste Behälterabschnitt weist eine verschließbare Behälteröffnung auf. Die Behälteröffnung des ersten Behälterabschnitts verbindet den ersten Behälterabschnitt und den zweiten Behälterabschnitt. Durch die Behälteröffnung kann Futter gravitätisch aus dem ersten Behälterabschnitt in den zweiten Behälterabschnitt gelangen. Vorzugsweise ist die Behälteröffnung angrenzend an ein unteres Ende der Trennwand angeordnet.

Die Behälteröffnung ist verschließbar, wobei ein Verschlusselement zum Verschließen der Behälteröffnung vorgesehen ist. Das Verschlusselement ist an der höhenverstellbaren Trennwand angeordnet, insbesondere an einem im Betriebszustand unterem Ende der höhenverstellbaren Trennwand. Das Verschlusselement kann vorzugsweise lösbar befestigt sein und/oder als Teil eines Minimaldosiermoduls nachgerüstet werden.

Die Trennwand ist zwischen dem ersten und den zweiten Behälterabschnitt angeordnet und höhenverstellbar ausgebildet. Die Trennwand kann somit vorzugsweise verschiedene Positionen einnehmen, z.B. eine obere und eine untere Position. Die Trennwand kann vorzugsweise stufenlos höhenverstellbar ausgebildet sein.

Eine Höhenverstellung der Trennwand bewirkt insbesondere eine Veränderung der Volumina des ersten und zweiten Behälterabschnitts und zwar derart, dass eine Höhenverstellung der Trennwand ein Volumen des ersten Behälterabschnitts vergrößert und ein Volumen des zweiten Behälterabschnitts verkleinert, und umgekehrt. Insbesondere führt eine Anhebung der Trennwand, also eine Höhenverstellung nach oben, zu einer Verkleinerung des Volumens des ersten Behälterabschnitts und zu einer Vergrößerung des Volumens des zweiten Behälterabschnitts, wohingegen ein Absenken der Trennwand, also eine Höhenverstellung nach unten, eine Vergrößerung des Volumens des ersten Behälterabschnitts und eine Verkleinerung des Volumens des zweiten Behälterabschnitts bewirkt.

Bei verschlossener Behälteröffnung wird ein durch den Volumendosierer bereitzustellendes Futtervolumen vorzugsweise bestimmt durch das Volumen des ersten Behälterabschnitts, welches von der Position der Trennwand abhängig ist. Bei geschlossener Behälteröffnung und einer oberen Position der Trennwand ist vorzugsweise das Futtervolumen für bereitzustellendes Futter minimal, wohingegen bei geschlossener Behälteröffnung und einer unteren Position der Trennwand vorzugsweise das Futtervolumen des bereitzustellenden Futters größer ist.

Bei geöffneter Behälteröffnung wird das Futtervolumen für vom Volumendosierer bereitzustellendes Futter vorzugsweise gebildet durch das Volumen des ersten Behälterabschnitts zuzüglich eines Teils des Volumens des zweiten Behälterabschnitts. Wie groß der Teil des zweiten Behälterabschnitts ist, der zum Futtervolumen für bereitzustellendes Futter zählt, hängt vorzugsweise ebenfalls von der Position der Trennwand ab. Eine obere Position der Trennwand führt vorzugsweise zu einem maximalen Futtervolumen für bereitzustellen Futter, da auf diese Weise ein Großteil des zweiten Behälterabschnitts zum Futtervolumen zählt. Eine untere Position der Trennwand führt bei geöffneter Behälteröffnung vorzugsweise zu einem kleineren Futtervolumen, da hier nur ein kleinerer Teil des zweiten Behälterabschnitts als Futtervolumen zur Verfügung steht.

Die höhenverstellbare Trennwand ist in einem Betriebszustand des Volumendosierers im Wesentlichen vertikal ausgerichtet. Ferner vorzugsweise sind der erste und der zweite Behälterabschnitt im Betriebszustand des Volumendosierers zumindest bereichsweise in horizontaler Richtung benachbart zueinander angeordnet.

Die Behälteröffnung und/oder der Futtereingang und/oder der Futterausgang sind vorzugsweise öffenbar verschließbar, können also von einer geöffneten Position in eine geschlossene Position und umgekehrt gebracht werden, wozu vorzugsweise geeignete Verschlussvorrichtungen vorgesehen sind.

Der hier beschriebene Volumendosierer beruht unter anderem auf der folgenden Erkenntnis: Beim Vorsehen von einem ersten einen zweiten Behälterabschnitt mit einer dazwischen angeordneten, höhenverstellbaren Trennwand, gelangt das Futter bei geöffneter Behälteröffnung durch den Futtereingang in den ersten Behälterabschnitt und von dort über die Behälteröffnung in den zweiten Behälterabschnitt, insbesondere in einen unteren Bereich des zweiten Behälterabschnitts, und füllt diesen bis etwa zur Höhe eines unteren Endes der höhenverstellbaren Trennwand. Weiteres Futter, welches über den Futtereingang in den ersten Behälterabschnitt gelangt, füllt dann den ersten Behälterabschnitt. Bei geöffneter Behälteröffnung ist somit das minimale Futtervolumen, das bereitgestellt werden kann, gebildet aus dem Volumen des ersten Behälterabschnitts sowie dem Volumen eines Teils des zweiten Behälterabschnitts, insbesondere eines unteren Teils des zweiten Behälterabschnitts, der bis zu einem unteren Ende der höhenverstellbaren Trennwand reicht. In bestimmten Anwendungsbereichen ist dieses Volumen nun jedoch noch zu groß und es werden deutlich kleinere Futtermengen gewünscht.

Durch das Vorsehen eines Verschlusselements, das die Behälteröffnung verschließt und an der höhenverstellbaren Trennwand, insbesondere an deren unteren Ende, angeordnet ist, kann die minimale Menge an auszudosierendem Futter reduziert werden. Durch das Verschließen der Behälteröffnung mit dem Verschlusselement steht in vorteilhafter Weise nur noch das Volumen des ersten Behälterabschnitts als Futtervolumen zur Verfügung. Durch eine Höhenverstellung der Trennwand und des daran befestigten Verschlusselements kann das Volumen des ersten Behälterabschnitts und damit auch das Futtervolumen verkleinert werden. Zur gravitätischen Abgabe des Futters ist dann nicht nur der Futterausgang, sondern auch die Behälteröffnung zu öffnen.

Der Volumendosierer ist somit bei geschlossener Behälteröffnung zur Dosierung sehr kleiner Futtervolumina geeignet, und bei geöffneter Behälteröffnung auch zur Dosierung größerer Volumina geeignet.

Der hier beschriebene Volumendosierer hat ferner den Vorteil einer robusten und wenig störungsanfälligen Konstruktion, die ferner eine vollständige Entleerung des Volumendosierers ermöglicht, ebenso wie eine gründliche Reinigung.

Der Futterbehälter kann einen Ausgabetrichter aufweisen. Vorzugsweise ist der Futterausgang an einem unteren Ende des Ausgabetrichters angeordnet. Ferner vorzugsweise ist der Ausgabetrichter lösbar befestigt, insbesondere an dem Futterbehälter, vorzugsweise an dem zweiten Behälterabschnitt. Der Ausgabetrichter kann vorzugsweise lösbar über eine Steck- Drehbewegung montiert und arretiert werden, beispielsweise über einen Bajonettverschluss. Die lösbare Befestigung des Ausgabetrichters erleichtert vorzugsweise die Reinigung und/oder Entleerung des Volumendosierers.

Es ist ferner bevorzugt, dass die Trennwand derart angeordnet ist, dass eine Höhenverstellung der Trennwand eine Füllhöhe des zweiten Behälterabschnitts bestimmt.

Die Trennwand, insbesondere deren unteres Ende, legt bei geöffneter Behälteröffnung vorzugsweise fest, bis zu welcher Höhe Futter, welches über den Futtereingang in den ersten Behälterabschnitt gelangt, in dem zweiten Behälterabschnitt gelangen kann.

Wenn der Futterausgang geschlossen ist und die Behälteröffnung geöffnet ist, kann über den Futtereingang Futter in den ersten Behälterabschnitt zugeführt werden. Durch die geöffnete Behälteröffnung fällt dieses Futter vorzugsweise so lange in einen unteren Bereich des zweiten Behälterabschnitts, bis die Trennwand, insbesondere deren unteres Ende, erreicht ist. Dann grenzt das im unteren Teil des zweiten Behälterabschnitts befindliche Futter vorzugsweise direkt an die Behälteröffnung an und verhindert den Durchtritt von weiterem Futter durch die Behälteröffnung. Weiteres zugeführtes Futter füllt somit anschließend den ersten Behälterabschnitt, bis vorzugsweise auch dieser gefüllt ist und kein weiteres Futter mehr durch den Futtereingang in den ersten Behälterabschnitt gelangen kann.

Insbesondere bestimmt eine Höhenverstellung der Trennwand vorzugsweise eine maximale Füllhöhe des zweiten Behälterabschnitts.

In einer bevorzugten Ausführungsvariante des Volumendosierer ist vorgesehen, dass das Verschlusselement als Verschlussklappe ausgebildet ist und/oder das Verschlusselement über einen Seilzug betätigbar ist und/oder das Verschlusselement nach unten oder zur Seite öffnet und/oder das Verschlusselement an einem unteren Ende der Trennwand angeordnet ist und/oder das Verschlusselement lösbar befestigt ist und/der das Verschlusselement durch Verschwenken um eine Schwenkachse öffenbar ist.

Vorzugsweise kann zur Betätigung des Verschlusselements und des Gewichtselements ein gemeinsamer Seilzug vorgesehen sein. Alternativ können das Verschlusselement und das Gewichtselement über separate Seilzüge betätigt werden.

Diese Ausgestaltungen des Verschlusselements haben den Vorteil einer robusten und zuverlässigen Lösung. Insbesondere bieten diese Ausgestaltungen den Vorteil, dass die Behälteröffnung durch das Verschlusselement sicher verschlossen werden kann, und auch durch den Gewichtsdruck von auf dem Verschlusselement lastenden Futter das Verschlusselement nicht ungewollt öffnet. Ferner wird beim Öffnen des Verschlusselements vorzugsweise die Behälteröffnung so weit freigegeben, dass eine vollständige Entleerung des ersten Behälterabschnitts gravitätisch erfolgen kann und insbesondere keine Futterreste im ersten Behälterabschnitt zurückbleiben. Eine zuverlässige Öffnung des Verschlusselements ist für eine zuverlässige Fütterung, insbesondere bei restriktiver Fütterung, von Vorteil.

Vorzugsweise ist das Verschlusselement an der Trennwand über eine lösbare Verbindung, wie beispielsweise eine Schnapp- und/oder Rast- und/oder Klickverbindung angeordnet. Vorzugsweise weist das Verschlusselement einen Vorsprung, beispielsweise einen Rasthaken, zum Eingriff in einer Ausnehmung der Trennwand auf. Ferner vorzugsweise ist eine Schwenkachse, um die das Verschlusselement, insbesondere die Verschlussklappe verschwenkt werden kann, im zweiten Behälterabschnitt angeordnet.

Eine bevorzugte Weiterbildung zeichnet sich dadurch aus, dass die höhenverstellbare Trennwand als erster Schenkel eines Kraftübertragungselements ausgebildet ist. Ferner ist bevorzugt, dass das Kraftübertragungselement einen zweiten Schenkel aufweist, der mit dem ersten Schenkel verbunden ist.

In einer weiteren bevorzugten Ausführungsform ist vorgesehen, dass an dem Kraftübertragungselement, insbesondere an dessen zweiten Schenkel, ein Einstellelement angeordnet ist.

Ferner ist vorzugsweise vorgesehen, dass der erste und der zweite Schenkel des Kraftübertragungselements über einen Verbindungsbogen miteinander verbunden sind. Dabei ist insbesondere bevorzugt, dass das Kraftübertragungselement derart ausgebildet ist, dass eine Höhenverstellung des ersten Schenkels eine entsprechend entgegengesetzte Höhenverstellung des zweiten Schenkels bewirkt.

Das Kraftübertragungselement dient insbesondere dazu, eine auf ein Einstellelement ausgeübte Kraft auf die Trennwand zu übertragen. Das Kraftübertragungselement ist dabei vorzugsweise ausgebildet, Schub- und/oder Zug- und/oder Druckkräfte zu übertragen.

Das Einstellelement, welches vorzugsweise an einem unteren Ende des zweiten Schenkels angeordnet ist, dient insbesondere dazu, ein gewünschtes Futtervolumen am Volumendosierer einzustellen. Hierzu kann das Einstellelement beispielsweise ein Anzeigeelement aufweisen, welches bei einer Höhenverstellung das eingestellte Volumen auf einer entsprechenden, am Volumendosierer angebrachten Volumenskala anzeigt. Beispielsweise kann ein Nutzer durch manuelles Verschieben des Einstellelements, insbesondere in vertikaler Richtung, das Futtervolumen verändern und vorzugsweise das mittels des Einstellelements eingestellte Futtervolumen über das Anzeigeelement auf der Volumenskala überprüfen. Vorzugsweise überträgt das Kraftübertragungselement die Bewegung des Einstellelements auf die Trennwand. Dies wird insbesondere dadurch ermöglicht, dass das Einstellelement am zweiten Schenkel des Kraftübertragungselements angeordnet ist und die Trennwand durch den ersten Schenkel des Kraftübertragungselements gebildet sind.

Die beiden Schenkel des Kraftübertragungselements sind vorzugsweise miteinander verbunden, insbesondere durch einen bogenförmigen Zwischenabschnitt, der auch als Verbindungsbogen bezeichnet werden kann. Das Kraftübertragungselement kann beispielsweise als streifenförmiges, gebogenes Element mit zwei im Wesentlichen geraden Schenkeln ausgebildet sein. Vorzugsweise verschiebt sich bei einer Höhenverstellung der Trennwand und/oder des Einstellelements entsprechend die jeweilige Länge der beiden Schenkel und die Position des Verbindungsbogens dazwischen. Vorzugsweise ist am Futterbehälter ein Führungselement zur Führung einer Bewegung, insbesondere der Höhenverstellung, des Kraftübertragungselements vorgesehen.

Ferner ist vorzugsweise vorgesehen, dass der zweite Schenkel des Kraftübertragungselements an einer Außenseite des Futterbehälters, insbesondere an einer Außenseite des zweiten Behälterabschnitts angeordnet ist.

Vorzugsweise ist das Kraftübertragungselement derart angeordnet, dass der erste Schenkel die Trennwand zwischen dem ersten und dem zweiten Behälterabschnitt bildet und der zweite Schenkel aus dem Futterbehälter hinausgeführt ist und an einer Außenseite des Futterbehälters, insbesondere an einer Außenseite des zweiten Behälterabschnitts, angeordnet ist. Der Verbindungsbogen zur Verbindung der beiden Schenkel des Kraftübertragungselements verläuft vorzugsweise in einem oberen Bereich des Volumendosierers, insbesondere des Futterbehälters oder auch an einer Außenseite des Volumendosierers, insbesondere des Futterbehälters.

Gemäß einer bevorzugten Ausführungsform ist vorgesehen, dass an dem Einstellelement ein Fixierelement vorgesehen ist zur Fixierung des zweiten Schenkels des Kraftübertragungselements und/oder des Einstellelements.

Das Fixierelement ist vorzugsweise, insbesondere in einer Richtung, beweglich ausgebildet. Das Fixierelement kann beispielsweise als Klemmelement ausgebildet sind und wirkt vorzugsweise mit einem Klemmkeil zusammen, der beispielsweise am Einstellelement ausgebildet sein kann, um den zweiten Schenkel des Kraftübertragungselements und/oder das Einstellelement zu fixieren. Diese Fixierung über ein Fixierelement ist insbesondere aus den folgenden Gründen vorteilhaft. Bei geschlossener Behälteröffnung kann im ersten Behälterabschnitt auf dem Verschlusselement befindliches Futter eine Gewichtskraft auf das Verschlusselement ausüben. Da das Verschlusselement an der höhenverstellbaren Trennwand angeordnet ist, überträgt sich diese Gewichtskraft auf die Trennwand. Bei einer leichtgängigen Höhenverstellung der Trennwand kann dies dazu führen, dass sich das Verschlusselement und damit die Trennwand durch die Gewichtskraft des Futters nach unten bewegen. Hierdurch könnte jedoch eine ungewollte Veränderung des vorangestellten Futtervolumens eintreten. Insbesondere um dies zu verhindern, können mit dem Fixierelement der zweite Schenkel des Kraftübertragungselements und/oder das Einstellelement, das an dem zweiten Schenkel des Kraftübertragungselements angeordnet ist, fixiert werden, beispielsweise an einer Außenseite des Behälters, insbesondere an einer Außenseite des zweiten Behälterabschnitts. Durch eine Fixierung des zweiten Schenkels des Kraftübertragungselements und/oder des Einstellelements kann somit in vorteilhafter Weise auch die Trennwand fixiert werden, die durch den ersten Schenkel des Kraftübertragungselements gebildet ist. Auf diese Weise wird dann auch die Position des Verschlusselements fixiert. Insbesondere ist das Fixierelement ausgebildet, den zweiten Schenkel des Kraftübertragungselements und/oder das Einstellelement gegen eine Bewegung zu sichern, die resultiert durch eine Gewichtskraft, die auf dem Verschlusselement befindliches Futter bewirkt. Das Fixierelement kann vorzugsweise lösbar ausgestaltet sein und/oder als Teil eines Minimaldosiermoduls nachgerüstet werden.

Vorzugsweise weist der Volumendosierer zusätzlich zu einer regulären Volumenskala eine Minimalvolumenskala auf. Die Minimalvolumenskala dient vorzugsweise dazu, die über ein Einstellelement einstellbaren Futtervolumina bei einer durch das Verschlusselement verschlossenen Behälteröffnung anzuzeigen. Dies ist insbesondere bevorzugt, wenn für die Einstellung der Futtervolumina bei geöffneter und geschlossener Behälteröffnung das gleiche Einstellelement verwendet werden soll. Je nachdem, ob die Behälteröffnung offen oder geschlossen ist, ergeben sich bei gleicher Position des Einstellelements unterschiedliche Futtervolumina. Daher ist es bevorzugt, neben einer regulären Volumenskala für die Verwendung des Volumendosierers mit geöffneter Behälteröffnung eine Minimalvolumenskala vorzusehen für die Verwendung des Volumendosierers mit geschlossener Behälteröffnung. Vorzugsweise ist die Minimalvolumenskala lösbar angeordnet und/oder optisch von der regulären Volumenskala unterscheidbar. Insbesondere kann es bevorzugt sein, dass auch die reguläre Volumenskala lösbar angeordnet ist. Beispielsweise kann ein Volumenskalaelement auf der Vorderseite mit der regulären Volumenskala und auf der Rückseite mit der Minimalvolumenskala versehen sein, sodass das Volumenskalaelement gelöst, gewendet und wieder befestigt werden kann, je nachdem ob der Volumendosierers mit geöffneter oder geschlossener Behälteröffnung verwendet werden soll.

Es ist ferner bevorzugt, dass der Futterbehälter einen Ausgabetrichter aufweist, an dessen unterem Ende der Futterausgang angeordnet ist. Der Ausgabetrichter ist vorzugsweise lösbar angeordnet, um eine Wartung und/oder Reinigung und/oder vollständige Entleerung des Futterbehälters zu gewährleisten. Der Ausgabetrichter ist vorzugsweise an einem unteren Ende des Futterbehälters angeordnet.

In einer bevorzugten Ausführungsform ist vorgesehen, dass der Volumendosierer ein Gewichtselement zum Verschließen des Futterausgangs aufweist, das vorzugsweise über einen Seilzug betätigbar ist. Das Gewichtselement kann beispielsweise als Gewichtskegel oder Gewichtskugel ausgebildet sein. Das Gewichtselement kann vorzugsweise über einen Seilzug angehoben werden, um den Futterausgang freizugeben, wobei ein Absenken des Gewichtselements über den Seilzug vorzugsweise zu einem Verschließen des Futterausgangs führt.

In einer weiteren bevorzugten Ausführungsform ist vorgesehen, dass der Futterbehälter und/oder der erste Behälterabschnitt und/oder der zweite Behälterabschnitt aus einem transparenten Material, insbesondere Kunststoff, bestehen oder dieses aufweisen.

Vorzugsweise sind der erste und der zweite Behälterabschnitt bis auf die höhenverstellbare Trennwand als einstückiges Bauteil ausgebildet. Eine einstückige Ausbildung kann die Herstellung von einzelnen Komponenten umfassen, die anschließend stoffschlüssig, beispielsweise durch Kleben und/oder Schweißen, miteinander verbunden werden. Ferner kann die einstückige Ausbildung die sequenzielle Anformung von Komponenten umfassen, beispielsweise die Herstellung eines ersten Bauteils und das Anspritzen eines zweiten Bauteils an das erste Bauteil. Besonders bevorzugt ist eine einstückige Ausbildung in dem Sinne, dass das einstückige Bauteil in einem Zug gefertigt wird, beispielsweise durch Spritzgießen. Das einstückige Bauteil besteht vorzugsweise durchgängig aus dem selben Material.

Eine weitere bevorzugte Fortbildung zeichnet sich dadurch aus, dass der Futterbehälter, insbesondere der erste Behälterabschnitt und/oder der zweite Behälterabschnitt, eine verschließbare Serviceöffnung aufweist. Die Serviceöffnung kann beispielsweise zur, beispielsweise manuellen oder automatischen, Zudosierung von Medikamenten und/oder Spezialfutter und/oder Futterzusatzstoffen genutzt werden. Die Serviceöffnung kann vorzugsweise auch zu Wartungs- und/oder Reinigungszwecken genutzt werden.

Ferner ist vorzugsweise vorgesehen, dass an einer Außenseite des Futterbehälters ein Befestigungselement angeordnet ist. Das Befestigungselement kann beispielsweise als Klammer, Clip oder dergleichen ausgebildet sein. Vorzugsweise dient das Befestigungselement dazu, Informationen, beispielsweise zu dem zu fütternden Nutztier, lösbar anzubringen.

Weitere vorteilhafte Ausführungsvarianten des zuvor beschriebenen Volumendosierers ergeben sich durch Kombination der hier erörterten bevorzugten Merkmale.

Gemäß einem weiteren Aspekt der Erfindung wird die eingangs genannte Aufgabe gelöst durch eine Fütterungsanlage nach Anspruch 12 zur Bereitstellung von Futter, insbesondere Trockenfutter für Schweine, insbesondere für die Einzeltierfütterung, umfassend eine Futterzuführleitung zur Bereitstellung von Futter an eine Vielzahl von Futtertrögen, wobei an einem, mehreren oder allen Futtertrögen einen Volumendosierer nach mindestens einem der vorhergehenden Ansprüche vorgesehen ist.

Vorzugsweise sind einer, mehrere oder alle Futtertröge der Fütterungsanlage über ein Fallrohr mit dem Futterausgang des Volumendosierers verbunden.

Gemäß einem weiteren Aspekt der Erfindung wird die eingangs genannte Aufgabe gelöst durch ein variables Volumendosiersystem nach Anspruch 13 für eine Fütterungsanlage zur Bereitstellung von Futter, insbesondere Trockenfutter für Schweine, insbesondere für die Einzeltierfütterung, das variable Volumendosiersystem umfassend einen Volumendosierer umfassend einen Futterbehälter mit einem ersten Behälterabschnitt mit einem Futtereingang und einem zweiten Behälterabschnitt mit einem verschließbaren Futterausgang zu gravitätischen Abgabe von Futter, wobei der erste Behälterabschnitt eine Behälteröffnung aufweist, welche den ersten Behälterabschnitt mit dem zweiten Behälterabschnitt verbindet für einen gravitätischen Durchtritt von Futter aus dem ersten Behälterabschnitt in den zweiten Behälterabschnitt wobei zwischen dem ersten und dem zweiten Behälterabschnitt eine höhenverstellbare Trennwand angeordnet ist, wobei eine Höhenverstellung der Trennwand ein Volumen des ersten Behälterabschnitts vergrößert und ein Volumen des zweiten Behälterabschnitts verkleinert, und umgekehrt, das variable Volumendosiersystem ferner umfassend Minimaldosiermodul aufweisend ein Verschlusselement, das ausgebildet ist, an der Trennwand angeordnet zu werden, um die Behälteröffnung zu verschließen.

Vorzugsweise weist das Minimaldosiermodul für ein variables Volumendosiersystem ein Fixierelement auf. Ferner vorzugsweise weist das Minimaldosiermodul eine Minimalvolumenskala auf.

Das Vorsehen eines Minimaldosiermoduls mit dem Verschlusselement für die Behälteröffnung und optional ferner mit einem Fixierelement und/oder eine Minimalvolumenskala hat den Vorteil, dass ein Volumendosierer nachträglich und/oder optional mit der Möglichkeit versehen werden kann, auch sehr kleine Futtervolumina zu dosieren. Verwendungsbereiche, in denen kleine Futtervolumina nicht erforderlich sind, kann der Volumendosierers ohne die Elemente des Minimaldosiermoduls ausgeliefert und verwendet werden. Für Anwendungsfälle, in denen sehr kleine Futtervolumina erforderlich sind, kann der Volumendosierer mit dem Minimaldosiermodul ausgeliefert und verwendet werden. Auf diese Weise ist auch eine Nachrüstung und/oder Umrüstung bereits im Einsatz befindliche Volumendosierer möglich.

Gemäß einem weiteren Aspekt der Erfindung wird die eingangs genannte Aufgabe gelöst durch ein Verfahren nach Anspruch 14 zur Bereitstellung von Futter, insbesondere Trockenfutter für Schweine, insbesondere für die Einzeltierfütterung, das Verfahren umfassend Verschließen eines an einem zweiten Behälterabschnitt eines Volumendosierers ausgebildeten Futterausgangs zur gravitätischen Abgabe von Futter, Verschließen einer Behälteröffnung für einen gravitätischen Durchtritt von Futter aus dem ersten Behälterabschnitt in den zweiten Behälterabschnitt, Höhenverstellung einer zwischen dem ersten und dem zweiten Behälterabschnitt angeordneten Trennwand, Übergeben von Futter durch einen Futtereingang in den ersten Behälterabschnitt, Öffnen der Behälteröffnung und des Futterausgangs.

Gemäß einem weiteren Aspekt der Erfindung wird die eingangs genannte Aufgabe gelöst durch ein Verfahren zum Variieren eines Volumendosiersystems, das Verfahren umfassend Bereitstellen eines Volumendosierers umfassend einen Futterbehälter mit einem ersten Behälterabschnitt mit einem Futtereingang und einem zweiten Behälterabschnitt mit einem verschließbaren Futterausgang zu gravitätischen Abgabe von Futter, wobei der erste Behälterabschnitt eine Behälteröffnung aufweist, welche den ersten Behälterabschnitt mit dem zweiten Behälterabschnitt verbindet für einen gravitätischen Durchtritt von Futter aus dem ersten Behälterabschnitt in den zweiten Behälterabschnitt wobei zwischen dem ersten und dem zweiten Behälterabschnitt eine höhenverstellbare Trennwand angeordnet ist, wobei eine Höhenverstellung der Trennwand ein Volumen des ersten Behälterabschnitts vergrößert und ein Volumen des zweiten Behälterabschnitts verkleinert, und umgekehrt, das Verfahren ferner umfassend Anordnen eines Verschlusselements eines Minimaldosiermoduls an der Trennwand zum Verschließen der Behälteröffnung

Vorzugsweise werden die Verfahrensschritte in der angegebenen Reihenfolge durchgeführt.

Die Fütterungsanlage, das variable Volumendosiersystem und die Verfahren sowie die jeweiligen möglichen Fortbildungen weisen vorzugsweise Merkmale bzw. Verfahrensschritte auf, die sie insbesondere dafür geeignet machen, für bzw. mit einem hier beschriebenen Volumendosierer und seinen Fortbildungen verwendet zu werden.

Zu den Vorteilen, Ausführungsvarianten und Ausführungsdetails dieser weiteren Aspekte der Erfindung und ihrer Fortbildungen wird auf die vorangegangene Beschreibung zu den entsprechenden Merkmalen des Volumendosierers bzw. der jeweiligen anderen Aspekte verwiesen.

Bevorzugte Ausführungsbeispiele werden beispielhaft anhand der beiliegenden Figuren beschrieben. Es zeigen:
- Figur 1:: eine dreidimensionale Ansicht eines Ausführungsbeispiels eines Volumendosierers;
- Figur 2:: den Volumendosierer gemäß Figur 1 im Längsschnitt;
- Figur 3:: einen Querschnitt des Volumendosierers gemäß Figur 1;
- Figur 4:: einen vergrößerten Ausschnitt aus Figur 2;
- Figur 5:: einen vergrößerten Ausschnitt aus Figur 3;
- Figuren 6a-d:: schematische Prinzipskizzen zur Veränderung der Futtervolumina bei geschlossener und geöffneter Behälteröffnung sowie bei verschiedenen Positionen der Trennwand; und
- Figur 7:: eine dreidimensionale Ansicht eines Ausführungsbeispiels eines Nutztierstalls mit einer Fütterungsanlage.

In den Figuren sind gleiche oder im Wesentlichen funktionsgleiche Elemente mit den gleichen Bezugszeichen versehen. Allgemeine Beschreibungen beziehen sich in der Regel auf alle Ausführungsformen, sofern Unterschiede nicht explizit angegeben sind

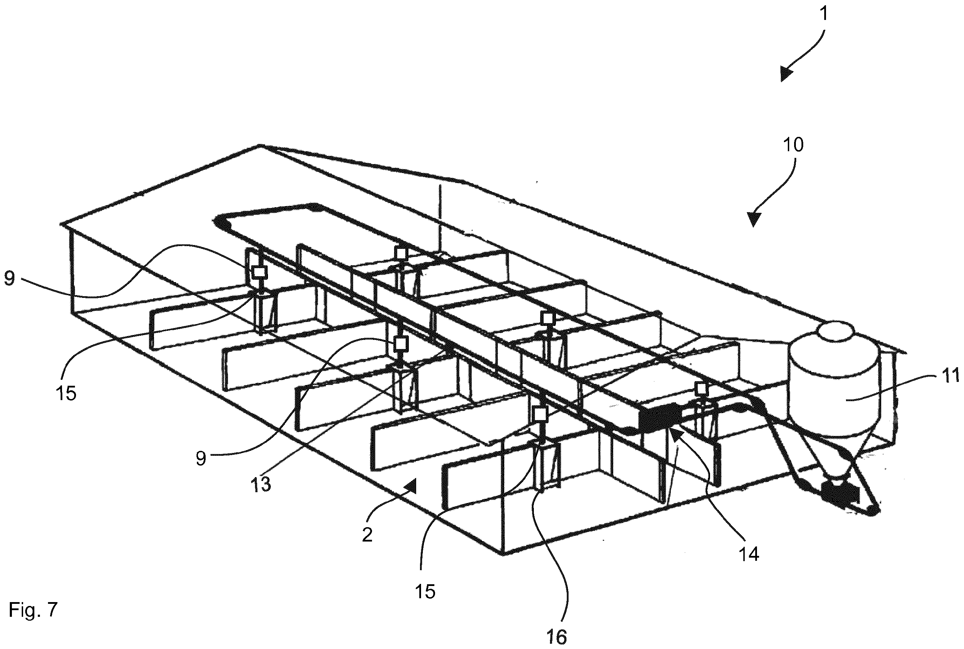

Figur 7 zeigt einen Nutztierstall 1, insbesondere einen Schweinestall, mit Abteilen 2 für die Gruppen- oder Einzeltierhaltung. Der Nutztierstall 1 weist eine Fütterungsanlage 10 auf zur Bereitstellung von Futter, insbesondere Trockenfutter für Schweine. Die Fütterungsanlage 10 weist eine Futterzuführleitung 13 auf zur Bereitstellung von Futter aus einem Futtervorrat 11 an eine Vielzahl von Futtertrögen 16. Die Futterzuführleitung 13 ist vorzugsweise als Förderrohr mit einer Förderkette mit Mitnehmerscheiben ausgebildet, welche über einen Antrieb 14 angetrieben wird, um Futter aus dem Futtervorrat 11 entlang der Futterzuführleitung 13 zu fördern. Das Futter gelangt gravitätisch von der Futterzuführleitung 13 über eine Vielzahl von Volumendosierern 9 über Fallrohre 15 in Futtertröge 16.

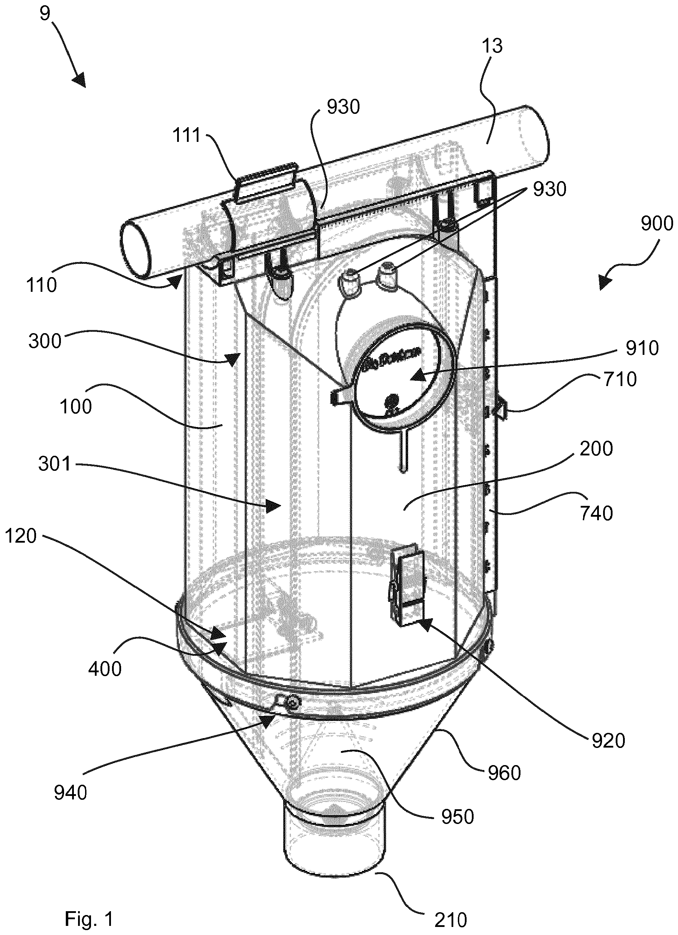

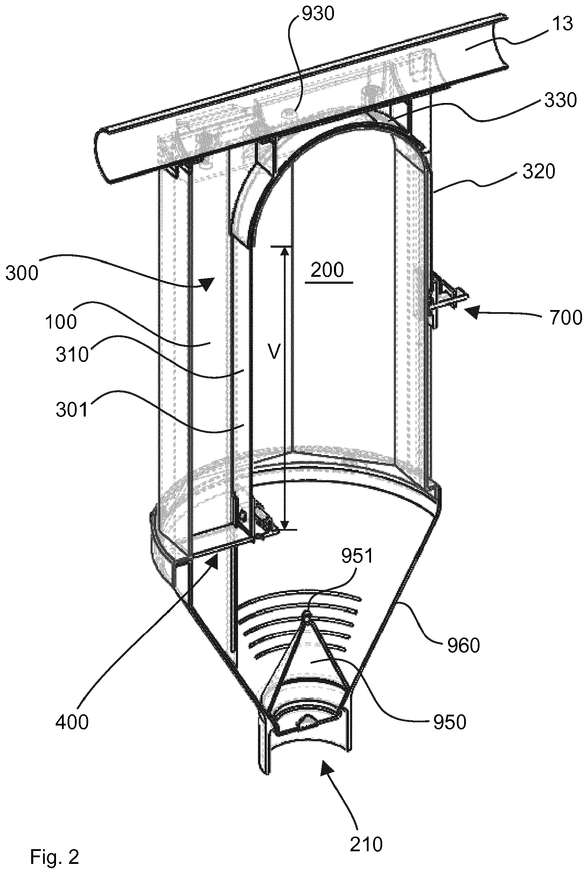

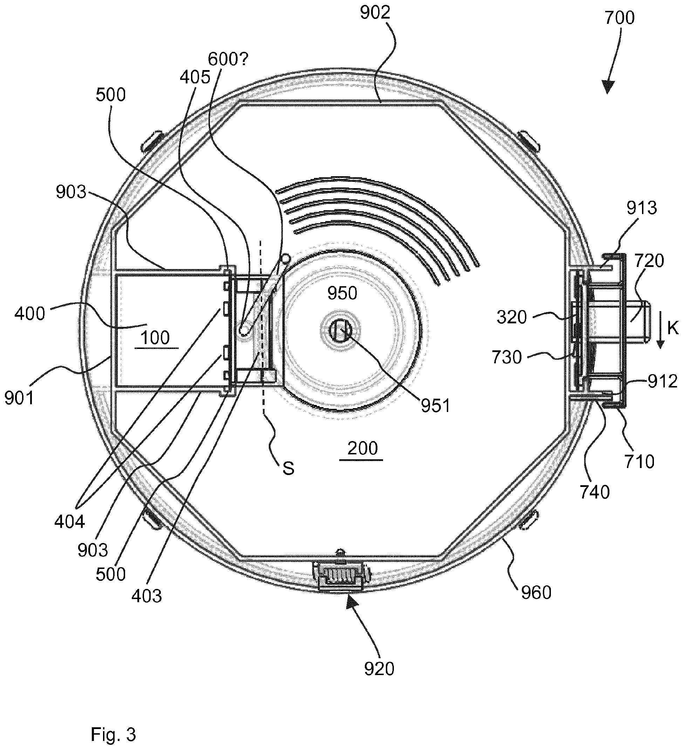

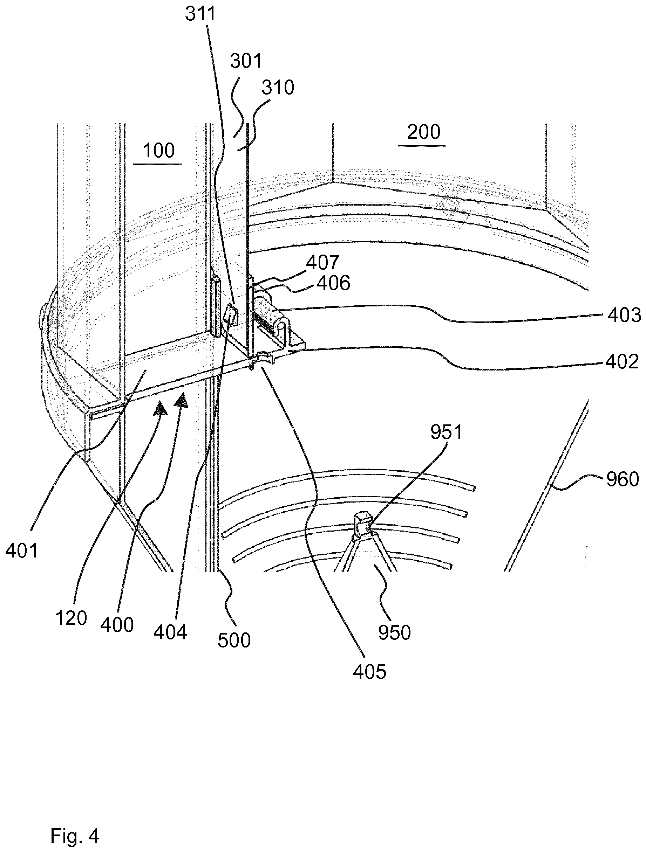

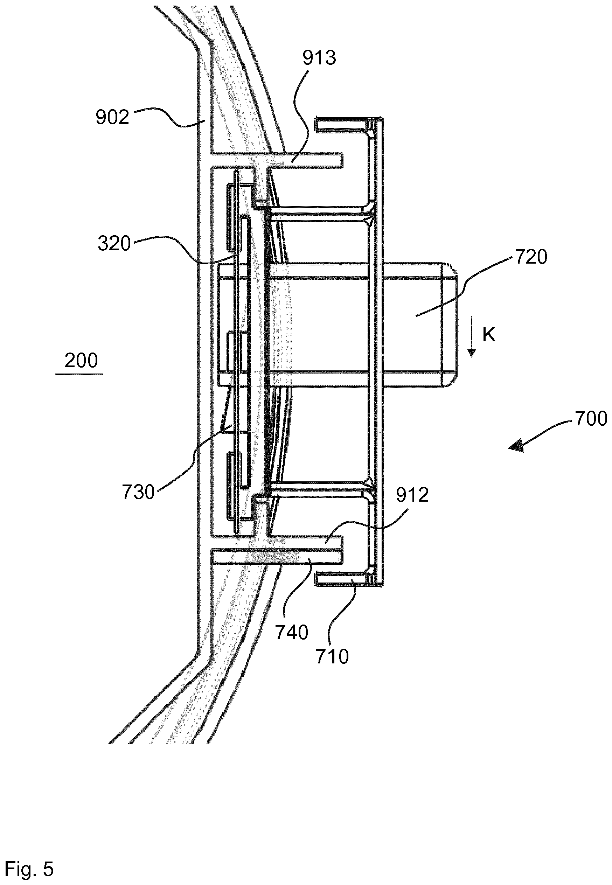

Der in den Figuren 1 bis 5 dargestellt Volumendosierer 9 für eine Fütterungsanlage 10 zur Bereitstellung von Futter weist einen Futterbehälter 900 mit einem Ausgabetrichter 960 auf. Der Ausgabetrichter 960 ist vorzugsweise über eine Steck-Dreh-Verbindung, beispielsweise einen Bajonettverschluss 940, an dem Futterbehälter 900, insbesondere dessen Außenwand 901, 902 lösbar befestigt. Diese lösbare Befestigung des Ausgabetrichters 960 trägt zur einfachen Wartung und/oder Reinigung und/oder vollständigen Entleerung des Volumendosierers 9 bei.

Der Futterbehälter 900 weist eine Serviceöffnung 910 auf, über die beispielsweise Futterzusatzstoffe in den Futterbehälter 900, insbesondere in den ersten Behälterabschnitt 100 und/oder in den zweiten Behälterabschnitt 200 hinzugefügt werden können. Ferner kann die Serviceöffnung 910 zur Wartung und/oder Reinigung des Volumendosierers 9 dienen.

Der Volumendosierer 9 weist ferner an einer Außenseite ein Befestigungselement 920 in Form einer Klammer auf. Das Befestigungselement 920 dient insbesondere dazu, Informationen zu dem zu fütternden Nutztier, vorzugsweise lösbar, befestigen zu können.

Der Futterbehälter 900 umfasst einen ersten Behälterabschnitt 100 und einen zweiten Behälterabschnitt 200, die in dem in den Figuren dargestellten Betriebszustand in horizontaler Richtung benachbart zueinander angeordnet sind.

Der erste Behälterabschnitt 100 weist einen Futtereingang 110 mit einem Schieber 111 auf. Der Futtereingang 110 dient zur Übergabe von Futter aus der Futterzuführleitung 13 in den Futterbehälter 900, insbesondere in den ersten Behälterabschnitt 100. Der erste Behälterabschnitt 100 weist an seinem unteren Ende eine verschließbare Behälteröffnung 120 auf, welche den ersten Behälterabschnitt 100 mit dem zweiten Behälterabschnitt 200 verbindet. Futter kann aus dem ersten Behälterabschnitt 100 durch die geöffnete Behälteröffnung 120 in den zweiten Behälterabschnitt 200, insbesondere einen unteren Bereich des Behälterabschnitts 200, gelangen.

Der zweite Behälterabschnitt 200 weist, insbesondere an dessen unteren Ende, einen Futterausgang 210 auf, der mit einem als Gewichtskegel ausgebildeten Gewichtselement 950 verschließbar ist. Das Gewichtselement 950 ist an seiner Spitze 951 mit einem nicht dargestellten Seilzug verbunden und über diesen betätigbar. Durch Anheben des Gewichtselements 950 per Seilzug wird der Futterausgang 210 zur gravitätischen Abgabe von Futter, beispielsweise an ein Fallrohr 15, freigegeben. Der Seilzug zur Betätigung des Gewichtselements 950 kann vorzugsweise über eine der Seilzugöffnungen 930 des Futterbehälters 900 nach außen geführt werden.

Zwischen dem ersten Behälterabschnitt 100 und dem zweiten Behälterabschnitt 200 ist eine höhenverstellbare Trennwand 301 angeordnet, die als erster Schenkel 310 eines Kraftübertragungselements 300 ausgebildet ist. Wenn hierin Eigenschaften oder Funktionen der höhenverstellbaren Trennwand beschrieben werden, gelten diese ebenso für das Kraftübertragungselement, insbesondere dessen ersten Schenkel, und umgekehrt.

Der erste Behälterabschnitt 100 ist ferner durch zwei weitere, ortsfeste bzw. unbewegliche Seitenwände 903 sowie eine Außenwand 901 begrenzt. Am unteren Ende des ersten Behälterabschnitts 100 ist die Behälteröffnung 120 angeordnet, durch die Futter gravitätisch aus dem ersten Behälterabschnitt 100 in den zweiten Behälterabschnitt 200 gelangen kann. Die Behälteröffnung ist hier somit durch die Außenwand 901, die ortsfesten Seitenwände 903 und die höhenverstellbare Trennwand 301 begrenzt. Hierdurch ergibt sich entlang der Höhe ein im Wesentlichen rechteckiger Querschnitt des Behälterabschnitts 100.

Auch der zweite Behälterabschnitt 200 ist begrenzt durch die weiteren ortsfesten Seitenwände 903 sowie ferner durch eine Außenwand 902. An den ortsfesten Seitenwänden 903 und/oder an dem Ausgabetrichter 960 sind Führungselemente in Form von Führungsschienen 500 zur Führung der höhenverstellbaren Trennwand 301 bzw. des ersten Schenkels 310 des Kraftübertragungselements 300 angeordnet.

An einem unteren Ende der höhenverstellbaren Trennwand 301 ist ein Verschlusselement 400 angeordnet. Das Verschlusselement 400 ist an einer Ausnehmung 311 am unteren Ende der höhenverstellbaren Trennwand 301 befestigt, insbesondere über eine Rastverbindung. Hierzu weist das Verschlusselement 400 einen Rastvorsprung 404 auf, der in die Ausnehmung 311 am unteren Ende der höhenverstellbaren Trennwand 301 rastend eingreift. Diese Verbindung hat den Vorteil einer einfachen (ggf. auch nachträglichen oder optionalen) Montage und Demontage des Verschlusselements bei gleichzeitig zuverlässiger Verbindung.

Das Verschlusselement 400 weist eine Verschlussklappe 401 auf, welche die Behälteröffnung 120 verschließt. Die Verschlussklappe 401 kann durch Verschwenken um eine im zweiten Behälterabschnitt 200 angeordnete Schwenkachse S nach unten auf geschwenkt werden, um die Behälteröffnung 120 freizugeben. Die Verschlussklappe 401 wird vorzugsweise über einen Seilzug 600, der an einer Seilöffnung 405 mit dem Verschlusselement 400 verbunden ist, in der geschlossenen Position gehalten durch eine Zugkraft. Bei Entspannen bzw. Freigabe des Seilzug 600 kann die Verschlussklappe 401 um die Schwenkachse S nach unten auch schwenken und so die Behälteröffnung 120 freigeben.

Das Verschlusselement 400 hat einem Vorsprung 402 in den zweiten Behälterabschnitt 200, und ist von dort aus über die Befestigungselemente 406, 407 mit dem Rastvorsprung 404 in der Ausnehmung 311 des ersten Schenkels 310 des Kraftübertragungselements 300 angeordnet. Die Verschlussklappe 401 ist über den Schwenkbügel 403 an der Schwenkachse S angelenkt. Die einfache und/oder lösbare Befestigung des Verschlusselements 400 ist insbesondere den Vorteil, wenn das Verschlusselement 400 Teil eines Minimaldosiermoduls ist, mit dem ein Volumendosierer optional und/oder nachträglich ausgerüstet werden kann.

An einem zweiten Schenkel 320 des Kraftübertragungselements 300 ist ein Einstellelement 700 angeordnet. Der erste und der zweite Schenkel 310, 320 des Kraftübertragungselements 300 sind über einen Verbindungsbogen 330 miteinander verbunden. Das Kraftübertragungselement 300 ist als streifenförmiges Element ausgebildet, beispielsweise als Blech und weist vorzugsweise ein rostfreies Material, wie beispielsweise rostfreier Stahl und/oder Kunststoff, auf oder besteht daraus.

Eine Höhenverstellung des ersten Schenkels 310 entlang des Verstellwegs V bewirkt eine entsprechend entgegengesetzte Höhenverstellung des zweiten Schenkels 320 und des daran angeordneten Einstellelements 700, und umgekehrt.

Das Einstellelement 700 weist ein Anzeigeelement 710 auf, welches auf einer Volumenskala 740 das eingestellte Futtervolumen anzeigt. Durch eine Höhenverstellung des Einstellelements 700 kann das eingestellte Futtervolumen verändert werden. Das Einstellelement 700 ist hierzu an dem zweiten Schenkel 320, insbesondere einem unteren Ende des zweiten Schenkels 320, angeordnet. Die Verbindung des Einstellelements 700 mit dem zweiten Schenkel 320 des Kraftübertragungselements 300 kann lösbar sein. Ferner kann die Verbindung vorzugsweise als Schnapp- und/oder Rast- und/oder Klickverbindung ausgebildet sein. Vorzugsweise weist das Einstellelement 700 einen Vorsprung auf zum Eingreifen in eine entsprechende Ausnehmung im zweiten Schenkel 320 des Kraftübertragungselements 300.

Über die Verbindung des Einstellelements 700 mit dem zweiten Schenkel 320 des Kraftübertragungselements bewirkt eine Höhenverstellung des Einstellelements 700 eine entsprechende, entgegengesetzte Höhenverstellung der Trennwand 301 und des daran angeordneten Verschlusselements 400. Umgekehrt kann, insbesondere bei einer leichtgängigen Höhenverstellung des Kraftübertragungselements 300, eine Positionsveränderung, insbesondere ein Absinken, des Verschlusselements 400 (bewirkt durch darauf befindliches Futter) eine ungewollte Veränderung des Futtervolumens und auch eine entsprechende Positionsveränderung des Einstellelements 700 verursachen. Um dies zu verhindern, weist das Einstellelement 700 ein Fixierelement 720 auf, das mit einem Klemmkeil 730 zusammenwirkt. Das Fixierelement 720 ist verschieblich gelagert und kann durch eine Bewegung in Fixierrichtung K und über ein Zusammenwirken mit dem Klemmkeil 730 das Einstellelement 700 und den zweiten Schenkel 320 des Kraftübertragungselements fixieren.

Der zweite Schenkel 320 des Kraftübertragungselement 300 ist an einer Außenseite des Futterbehälters 900, insbesondere an einer Außenseite der Außenwand 902 des zweiten Futterbehälters 200 angeordnet. Der Verbindungsbogen 330 des Kraftübertragungselements 300 verläuft an einem oberen Ende des Volumendosierers bzw. in einem oberen Bereich des zweiten Behälterabschnitts 200.

An einem Vorsprung 912 der Außenwand 902 des zweiten Behälterabschnitts 200 ist eine Volumenskala 740 angeordnet. Auf dieser Volumenskala 740 kann das mittels des Einstellelements 700 eingestellte Futtervolumen über das Anzeigeelement 710 abgelesen werden. Die Volumenskala 740 ist vorzugsweise lösbar befestigt und kann beispielsweise auch auf dem gegenüberliegenden Vorsprung 913 angeordnet werden, beispielsweise bei einer veränderten Einbausituation.

Es ist bevorzugt, eine Minimalvolumenskala bereitzustellen, welche zusätzlich oder anstelle der regulären Volumenskala 740 eingesetzt werden kann, wenn der Volumendosierer zum Dosieren von minimalen Futtervolumina mit geschlossener Behälteröffnung 120 eingesetzt werden soll. Bei geschlossener Behälteröffnung 120 ergibt sich für eine bestimmte Position des Einstellelements 700 ein anderes Futtervolumen als bei geöffneter Behälteröffnung. Daher ist es von Vorteil, wenn über eine zusätzliche Minimalvolumenskala auch die Minimalfuttervolumina bei der Verwendung mit geschlossener Behälteröffnung 120 abgelesen werden kann.

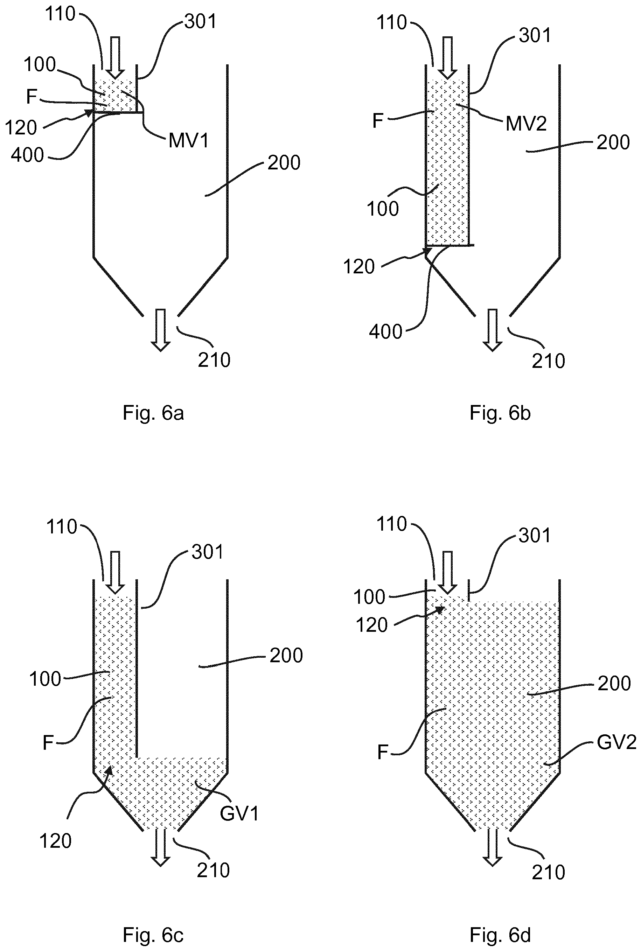

In den Figuren 6a bis 6d ist anhand von schematischen Prinzipskizzen die Dosierung vereinfacht dargestellt. Die Figuren 6a und 6b zeigen die Verwendung des Volumendosierers mit durch das Verschlusselement 400 verschlossener Behälteröffnung 120 und die Figuren 6c und 6d die Verwendung mit geöffneter Behälteröffnung 120. Zur vereinfachten Darstellung ist in den Figuren 6c und 6d das Verschlusselement 400 nicht dargestellt. Insgesamt sind in den Prinzipskizzen 6a bis 6d verschiedene Komponenten des Volumendosierers nicht dargestellt.

Figur 6a zeigt die Trennwand 301 in einer oberen Position sowie die durch das Verschlusselement 400 geschlossener Behälteröffnung 120. Auf diese Weise ergibt sich ein minimales Volumen des ersten Behälterabschnitts 100 und ein maximales Volumen des zweiten Behälterabschnitts 200. Da die Behälteröffnung 120 durch das Verschlusselement geschlossen ist, kann über den Futtereingang 110 in den ersten Behälterabschnitt 100 gelangen des Futter F nur das Minimalvolumen MV1 füllen. Auf diese Weise ist auch die Dosierung einer sehr kleinen Futtermenge möglich. Zum Fütterungszeitpunkt werden dann das Verschlusselement 400 und der Futterausgang 210 geöffnet, sodass das Futter F gravitätisch austreten kann.

Im Figur 6b ist die Trennwand 301 in einer unteren Position dargestellt. Die Behälteröffnung 120 des ersten Behälterabschnitts 100 ist wieder durch das Verschlusselement 400 verschlossen. Hier ergibt sich als Futtervolumen MV2 nun das maximale Volumen des ersten Behälterabschnitts 100, das Volumen des zweiten Behälterabschnitts 200 ist entsprechend verkleinert. Wird nun das Verschlusselement 400 sowie der Futterausgang 210 geöffnet, kann das Futter F der Menge MV2 gravitätisch austreten.

Figur 6c zeigt die Trennwand 301 ebenfalls in der unteren Position, jedoch mit geöffneter Behälteröffnung 120. Über den Futtereingang 110 in den ersten Behälterabschnitt 100 Eintreten des Futter F gelangt durch die geöffnete Behälteröffnung 120 in einem unteren Bereich des zweiten Behälterabschnitts 200 und füllt diesen (bei geschlossenem Futterausgang 210) etwa bis zur Höhe eines unteren Endes der Trennwand 301. Das im unteren Teil des zweiten Behälterabschnitts 200 befindliche Futter blockiert sodann die Behälteröffnung 120, sodass weiteres über den Futtereingang 110 zugeführtes Futter im ersten Behälterabschnitt 100 verbleibt. Das hier dosierte Futtervolumen GV1 ist deutlich größer als das Minimalvolumen MV1 aus Figur 6a und auch größer als das Futtervolumen MV2 aus Figur 6b. Durch Öffnen des Futterausgangs 210 kann das Futtervolumen GV1 gravitätisch austreten.

In Figur 6d ist die Trennwand 301 in einer oberen Position dargestellt und die Behälteröffnung 120 geöffnet. In dieser Situation ergibt sich das maximale Futtervolumen GV2. Auch hier tritt Futter F durch den Futtereingang 110 in den ersten Behälterabschnitt 100 ein und von dort durch die Behälteröffnung 120 in den zweiten Behälterabschnitt 200 und füllt diesen vom geschlossenen Futterausgang 210 aus beginnend bis zu einem unteren Ende der Trennwand 301. Da sich die Trennwand 301 in einer oberen Position befindet, wird der zweite Behälterabschnitt 200 nahezu vollständig mit Futter F befüllt. Weiteres Futter kann dann noch in den (hier aufgrund der oberen Position der Trennwand 301 nur kleinen) Behälterabschnitt 100 nachgefüllt werden, um das Gesamt-Maximalvolumen GV2 zu bilden. Auch dieses maximale Futtervolumen GV2 kann durch Öffnen des Futterausgangs 210 gravitätisch ausgegeben werden.

Der hier beschriebene Volumendosierer ermöglicht es somit in vorteilhafter Weise, nicht nur verschieden große Futtervolumina einzustellen und ausdosieren zu können, sondern auch, insbesondere durch das Vorsehen eines Verschlusselements 400, den ersten Behälterabschnitt 100 auch zur Einstellung und Ausdosierung von deutlich kleineren Futtermengen nutzen zu können. Auf diese Weise kann der Einsatzbereich des Volumendosierers vergrößert werden. Gleichzeitig ermöglicht die hier beschriebene Konstruktion des Volumendosierers einen robusten und zuverlässigen Betrieb, was in der Nutztierhaltung, gerade auch für das Tierwohl, von besonderer Bedeutung ist.

### BEZUGSZEICHENLISTE

- 1: Nutztierstall
- 2: Abteil
- 9: Volumendosierer
- 10: Fütterungsanlage
- 11: Futtervorrat
- 13: Futterzuführleitung
- 14: Antrieb
- 15: Fallrohr
- 16: Futtertrog

- 100: ersten Behälterabschnitt
- 110: Futtereingang
- 111: Schieber
- 120: Behälteröffnung

- 200: zweitem Behälterabschnitt
- 210: Futterausgang

- 300: Kraftübertragungselement
- 301: höhenverstellbare Trennwand
- 310: erster Schenkel
- 311: Ausnehmung
- 320: zweiter Schenkel
- 330: Verbindungsbogen

- 400: Verschlusselement
- 401: Verschlussklappe
- 402: Vorsprung
- 403: Schwenkbügel
- 404: Rastvorsprung
- 405: Seilöffnung
- 406,407: Befestigungselemente

- 500: Führungsschienen
- 600: Seilzug
- 700: Einstellelement
- 710: Anzeigeelement
- 720: Fixierelement
- 730: Klemmkeil
- 740: Volumenskala

- 900: Futterbehälter
- 901,902: Außenwand
- 903: ortsfeste Seitenwände
- 910: Serviceöffnungen
- 912, 913: Vorsprung der Außenwand
- 920: Befestigungselement
- 930: Seilzugöffnungen
- 940: Bajonettverschluss
- 950: Gewichtselement
- 951: Spitze des Gewichtselements
- 960: Ausgabetrichter

- F: Futter
- K: Fixierrichtung
- S: Schwenkachse
- MV1: Minimalvolumen
- MV2, GV1: Futtervolumen
- GV2: Maximalvolumen

## Patentansprüche

1. Volumendosierer (9) für eine Fütterungsanlage (10) zur Bereitstellung von Futter, insbesondere Trockenfutter für Schweine, insbesondere für die Einzeltierfütterung, der Volumendosierer umfassend
- einen Futterbehälter (900) mit einem ersten Behälterabschnitt (100) mit einem Futtereingang (110) und einem zweiten Behälterabschnitt (200) mit einem verschließbaren Futterausgang (210) zur gravitätischen Abgabe von Futter (F),
- wobei der erste Behälterabschnitt (100) eine verschließbare Behälteröffnung (120) aufweist, welche den ersten Behälterabschnitt (100) mit dem zweiten Behälterabschnitt (200) verbindet für einen gravitätischen Durchtritt von Futter (F) aus dem ersten Behälterabschnitt (100) in den zweiten Behälterabschnitt (200),
- wobei zwischen dem ersten (100) und dem zweiten Behälterabschnitt (200) eine höhenverstellbare Trennwand (301) angeordnet ist, wobei die höhenverstellbare Trennwand (301) in einem Betriebszustand des Volumendosierers im Wesentlichen vertikal ausgerichtet ist,
- **dadurch gekennzeichnet, dass** an der Trennwand (301) ein Verschlusselement (400) zum Verschließen der Behälteröffnung (120) angeordnet ist, und
dass zur gravitätischen Abgabe des Futters aus dem ersten Behälterabschnitt nicht nur der Futterausgang (210), sondern auch die Behälteröffnung (120) zu öffnen ist.

2. Volumendosierer (9) nach mindestens einem der vorhergehenden Ansprüche,
wobei die Trennwand derart angeordnet ist, dass eine Höhenverstellung der Trennwand (301) eine Füllhöhe des zweiten Behälterabschnitts (200) bestimmt.

3. Volumendosierer (9) nach mindestens einem der vorhergehenden Ansprüche,
wobei das Verschlusselement (400) als Verschlussklappe (401) ausgebildet ist und/oder das Verschlusselement (400) über einen Seilzug (600) betätigbar ist und/oder das Verschlusselement (400) nach unten oder zur Seite öffnet und/oder das Verschlusselement (400) an einem unteren Ende der Trennwand angeordnet ist und/oder das Verschlusselement (400) lösbar befestigt ist und/der das Verschlusselement durch Verschwenken um eine Schwenkachse (S) öffenbar ist.

4. Volumendosierer (9) nach mindestens einem der vorhergehenden Ansprüche,
wobei die höhenverstellbare Trennwand (301) als erster Schenkel eines Kraftübertragungselements ausgebildet ist.

5. Volumendosierer nach dem vorhergehenden Anspruch,
wobei das Kraftübertragungselement (300) einen zweiten Schenkel (320) aufweist, der mit dem ersten Schenkel (310) verbunden ist.

6. Volumendosierer (9) nach mindestens einem der beiden vorhergehenden Ansprüche,
wobei an dem Kraftübertragungselement (300), insbesondere an dessen zweiten Schenkel (320) ein Einstellelement (700) angeordnet ist.

7. Volumendosierer (9) nach dem vorhergehenden Anspruch,
wobei der erste (310) und der zweite Schenkel (320) des Kraftübertragungselements (300) über einen Verbindungsbogen (330) miteinander verbunden sind, und/oder
wobei das Kraftübertragungselement (300) derart ausgebildet ist, dass eine Höhenverstellung des ersten Schenkels (310) eine entsprechend entgegengesetzte Höhenverstellung des zweiten Schenkels (320) bewirkt.

8. Volumendosierer (9) nach mindestens einem der beiden vorhergehenden Ansprüche,
wobei der zweite Schenkel (320) des Kraftübertragungselements (300) an einer Außenseite des Futterbehälters (900), insbesondere an einer Außenseite des zweiten Behälterabschnitts (200), angeordnet ist.

9. Volumendosierer (9) nach Anspruch 6,
wobei an dem Einstellelement ein Fixierelement (720) vorgesehen ist zur Fixierung des zweiten Schenkels des Kraftübertragungselements (300) und/oder des Einstellelements.

10. Volumendosierer (9) nach mindestens einem der vorhergehenden Ansprüche,
wobei der Futterbehälter (900) einen Ausgabetrichter (960) aufweist, an dessen unterem Ende der Futterausgang (210) angeordnet ist.

11. Volumendosierer (9) nach mindestens einem der vorhergehenden Ansprüche,
wobei der Volumendosierer ein Gewichtselement (950) zum Verschließen des Futterausgangs (210) aufweist.

12. Fütterungsanlage (10) zur Bereitstellung von Futter, insbesondere Trockenfutter für Schweine, insbesondere für die Einzeltierfütterung, umfassend eine Vielzahl von Futtertrögen (16) und eine Futterzuführleitung (13) zur Bereitstellung von Futter an die Vielzahl von Futtertrögen (16), wobei an einem, mehreren oder allen Futtertrögen (16)ein Volumendosierer (9) nach mindestens einem der vorhergehenden Ansprüche vorgesehen ist.

13. Variables Volumendosiersystem für eine Fütterungsanlage (10) zur Bereitstellung von Futter, insbesondere Trockenfutter für Schweine, insbesondere für die Einzeltierfütterung, das variable Volumendosiersystem umfassend
einen Volumendosierer (9) umfassend
- einen Futterbehälter (900) mit einem ersten Behälterabschnitt (100) mit einem Futtereingang (110) und einem zweiten Behälterabschnitt (200) mit einem verschließbaren Futterausgang (210) zu gravitätischen Abgabe von Futter,
- wobei der erste Behälterabschnitt (100) eine Behälteröffnung (120) aufweist, welche den ersten Behälterabschnitt (100) mit dem zweiten Behälterabschnitt (200) verbindet für einen gravitätischen Durchtritt von Futter aus dem ersten Behälterabschnitt (100) in den zweiten Behälterabschnitt (200),
- wobei zwischen dem ersten (100) und dem zweiten Behälterabschnitt (200) eine höhenverstellbare Trennwand (301) angeordnet ist, wobei die höhenverstellbare Trennwand in einem Betriebszustand des Volumendosierers im Wesentlichen vertikal ausgerichtet ist, - wobei
eine Höhenverstellung der Trennwand ein Volumen des ersten Behälterabschnitts (100) vergrößert und ein Volumen des zweiten Behälterabschnitts (200) verkleinert, und umgekehrt,
**dadurch gekennzeichnet, dass** und zur gravitätischen Abgabe des Futters aus dem ersten Behälterabschnitt nicht nur der Futterausgang, sondern auch die Behälteröffnung zu öffnen ist,
das variable Volumendosiersystem ferner umfassend Minimaldosiermodul aufweisend ein Verschlusselement, das ausgebildet ist, an der Trennwand angeordnet zu werden, um die Behälteröffnung (120) zu verschließen.

14. Verfahren zur Bereitstellung von Futter, insbesondere Trockenfutter für Schweine, insbesondere für die Einzeltierfütterung,
das Verfahren umfassend:
- Verschließen eines an einem zweiten Behälterabschnitt (200) eines Futterbehälters (900) eines Volumendosierers (9) ausgebildeten Futterausgangs (210) zur gravitätischen Abgabe von Futter,
- Verschließen einer Behälteröffnung (120) für einen gravitätischen Durchtritt von Futter aus einem ersten Behälterabschnitt (100) in den zweiten Behälterabschnitt (200) durch ein Verschlusselement (400), das an einer zwischen dem ersten (100) und dem zweiten Behälterabschnitt (200) angeordneten Trennwand angeordnet ist,
- Höhenverstellung der Trennwand (301), wobei die höhenverstellbare Trennwand in einem Betriebszustand des Volumendosierers im Wesentlichen vertikal ausgerichtet ist,
- Übergeben von Futter durch einen Futtereingang (110) in den ersten Behälterabschnitt (100),
- Öffnen der Behälteröffnung (120) und des Futterausgangs (210) zur gravitätischen Abgabe des Futters aus dem ersten Behälterabschnitt.

15. Verfahren zum Variieren eines Volumendosiersystems, das Verfahren umfassend:
Bereitstellen eines Volumendosierers umfassend
- einen Futterbehälter (900) mit einem ersten Behälterabschnitt (100) mit einem Futtereingang und einem zweiten Behälterabschnitt (200) mit einem verschließbaren Futterausgang (210) zu gravitätischen Abgabe von Futter,
- wobei der erste Behälterabschnitt (100) eine Behälteröffnung (120) aufweist, welche den ersten Behälterabschnitt (100) mit dem zweiten Behälterabschnitt (200) verbindet für einen gravitätischen Durchtritt von Futter aus dem ersten Behälterabschnitt (100) in den zweiten Behälterabschnitt (200),
- wobei zwischen dem ersten (100) und dem zweiten Behälterabschnitt (200) eine höhenverstellbare Trennwand (301) angeordnet ist, wobei die höhenverstellbare Trennwand in einem Betriebszustand des Volumendosierers im Wesentlichen vertikal ausgerichtet ist, und wobei zur gravitätischen Abgabe des Futters aus dem ersten Behälterabschnitt nicht nur der Futterausgang, sondern auch die Behälteröffnung zu öffnen ist,
- wobei eine Höhenverstellung der Trennwand (301) ein Volumen des ersten Behälterabschnitts (100) vergrößert und ein Volumen des zweiten Behälterabschnitts (200) verkleinert, und umgekehrt,
das Verfahren ferner umfassend
- Anordnen eines Verschlusselements (400) eines Minimaldosiermoduls an der Trennwand zum Verschließen der Behälteröffnung (120).

## Claims

1. Volumetric doser (9) for a feeding installation (10) for providing feed, in particular dry feed for pigs, in particular for feeding individual animals, the volumetric doser comprising
- a feed container (900) having a first container section (100) with a feed inlet (110) and a second container section (200) with a closable feed outlet (210) for gravitational dispensing of feed (F),
- wherein the first container section (100) has a closable container opening (120) which connects the first container section (100) to the second container section (200) for a gravitational passage of feed (F) from the first container section (100) into the second container section (200),
- wherein a height-adjustable partition wall (301) is arranged between the first (100) and the second container section (200), wherein the height-adjustable partition wall (301) is oriented substantially vertically in an operating state of the volumetric doser,
- **characterized in that** a closure element (400) for closing the container opening (120) is arranged on the partition wall (301), and
that not only the feed outlet (210) but also the container opening (120) is to be opened for gravitational dispensing of the feed from the first container section.

2. Volumetric doser (9) according to at least one of the preceding claims,
wherein the partition wall is arranged in such a way that a height adjustment of the partition wall (301) determines a filling height of the second container section (200).

3. Volumetric doser (9) according to at least one of the preceding claims,
wherein the closure element (400) is designed as a closure flap (401) and/or the closure element (400) can be actuated via a cable pull (600) and/or the closure element (400) opens downwards or to the side and/or the closure element (400) is arranged at a lower end of the partition wall and/or the closure element (400) is detachably fastened and/or the closure element is openable by pivoting about a pivot axis (S).

4. Volumetric doser (9) according to at least one of the preceding claims,
wherein the height-adjustable partition wall (301) is designed as the first leg of a force transmission element.

5. Volumetric doser according to the preceding claim,
wherein the force transmission element (300) has a second leg (320) which is connected to the first leg (310).

6. Volumetric doser (9) according to at least one of the two preceding claims,
wherein an adjusting element (700) is arranged on the force transmission element (300), in particular on its second leg (320).

7. Volumetric doser (9) according to the preceding claim,
wherein the first (310) and the second leg (320) of the force transmission element (300) are connected to one another via a connecting arc (330), and/or wherein the force transmission element (300) is designed in such a way that a height adjustment of the first leg (310) causes a correspondingly opposite height adjustment of the second leg (320).

8. Volumetric doser (9) according to at least one of the two preceding claims,
wherein the second leg (320) of the force transmission element (300) is arranged on an outer side of the feed container (900), in particular on an outer side of the second container section (200).

9. Volumetric doser (9) according to claim 6,
wherein a fixing element (720) is provided on the adjusting element for fixing the second leg of the force transmission element (300) and/or the adjusting element.

10. Volumetric doser (9) according to at least one of the preceding claims,
wherein the feed container (900) has a dispensing hopper (960), at the lower end of which the feed outlet (210) is arranged.

11. Volumetric doser (9) according to at least one of the preceding claims,
wherein the volumetric doser has a weight element (950) for closing the feed outlet (210).

12. Feeding installation (10) for providing feed, in particular dry feed for pigs, in particular for feeding individual animals,
comprising a plurality of feed troughs (16) and a feed supply line (13) for providing feed to the plurality of feed troughs (16), wherein a volumetric doser (9) according to at least one of the preceding claims is provided on one, several or all feed troughs (16).

13. Variable volumetric dosing system for a feeding installation (10) for providing feed, in particular dry feed for pigs, in particular for feeding individual animals, the variable volume dosing system comprising
a volumetric doser (9) comprising
- a feed container (900) having a first container section (100) with a feed inlet (110) and a second container section (200) with a closable feed outlet (210) for gravitational dispensing of feed,
- wherein the first container section (100) has a container opening (120) which connects the first container section (100) to the second container section (200) for gravitational passage of feed from the first container section (100) into the second container section (200),
- wherein a height-adjustable partition wall (301) is arranged between the first (100) and the second container section (200), wherein the height-adjustable partition wall is oriented substantially vertically in an operating state of the volumetric doser,
- wherein a height adjustment of the partition wall increases a volume of the first container section (100) and reduces a volume of the second container section (200), and vice versa,
**characterized in that** for gravitational dispensing of the feed from the first container section not only the feed outlet but also the container opening is to be opened,
the variable volumetric dosing system further comprising a minimum dosing module having a closure element which is designed to be arranged on the partition wall to close the container opening (120).

14. Method for providing feed, in particular dry feed for pigs, in particular for feeding individual animals,
the method comprising:
- Closure of a feed outlet (210) formed on a second container section (200) of a feed container (900) of a volumetric doser (9) for gravitational dispensing of feed,
- Closure of a container opening (120) for a gravitational passage of feed from a first container section (100) into the second container section (200) by a closure element (400) which is arranged on a partition wall arranged between the first (100) and the second container section (200),
- Height adjustment of the partition wall (301), wherein the height-adjustable partition wall is oriented substantially vertically in an operating state of the volumetric doser,
- Transfer of feed through a feed inlet (110) into the first container section (100),
- Opening of the container opening (120) and the feed outlet (210) for gravitational dispensing of the feed from the first container section.

15. Method for varying a volumetric dosing system, the method comprising:
Provision of a volumetric doser comprising
- a feed container (900) having a first container section (100) with a feed inlet and a second container section (200) with a closable feed outlet (210) for gravitational dispensing of feed,
- wherein the first container section (100) has a container opening (120) which connects the first container section (100) to the second container section (200) for gravitational passage of feed from the first container section (100) into the second container section (200),
- wherein a height-adjustable partition wall (301) is arranged between the first (100) and the second container section (200), wherein the height-adjustable partition wall is arranged substantially vertically in an operating state of the volumetric doser, and wherein not only the feed outlet but also the container opening is to be opened for gravitational dispensing of the feed from the first container section,
- wherein a height adjustment of the partition wall (301) increases a volume of the first container section (100) and reduces a volume of the second container section (200), and vice versa,
the method further comprising
- Arrangement of a closure element (400) of a minimum dosing module on the partition wall for closing the container opening (120).

## Revendications

1. Doseur volumétrique (9) pour une installation d'alimentation pour animaux (10) pour la mise à disposition d'aliments pour animaux, en particulier d'aliments secs pour cochons, en particulier pour l'alimentation individuelle des animaux, le doseur volumétrique comprenant
- un récipient d'aliments pour animaux (900) avec une première section de récipient (100) avec une entrée d'aliments pour animaux (110) et une deuxième section de récipient (200) avec une sortie d'aliments pour animaux (210) pouvant être fermée pour la distribution par gravité d'aliments pour animaux (F),
- dans lequel la première section de récipient (100) présente une ouverture de récipient (120) pouvant être fermée, laquelle relie la première section de récipient (100) à la deuxième section de récipient (200) pour un passage par gravité d'aliments pour animaux (F) de la première section de récipient (100) à la deuxième section de récipient (200),
- dans lequel une cloison de séparation réglable en hauteur (301) est agencée entre la première (100) et la deuxième section de récipient (200), dans lequel la cloison de séparation réglable en hauteur (301) est orientée sensiblement à la verticale dans un état de fonctionnement du doseur volumétrique,
- **caractérisé en ce qu'**un élément de fermeture (400) pour la fermeture de l'ouverture de récipient (120) est agencé au niveau de la cloison de séparation (301), et
**en ce que** pour la distribution par gravité des aliments pour animaux à partir de la première section de récipient, il faut ouvrir non seulement la sortie d'aliments pour animaux (210) mais aussi l'ouverture de récipient (120).

2. Doseur volumétrique (9) selon au moins l'une quelconque des revendications précédentes,
dans lequel la cloison de séparation est agencée de telle sorte qu'un réglage en hauteur de la cloison de séparation (301) détermine une hauteur de remplissage de la deuxième section de récipient (200).

3. Doseur volumétrique (9) selon au moins l'une quelconque des revendications précédentes,
dans lequel l'élément de fermeture (400) est réalisé sous forme de clapet de fermeture (401) et/ou l'élément de fermeture (400) peut être actionné par le biais d'un câble de traction (600) et/ou l'élément de fermeture (400) s'ouvre vers le bas ou sur le côté et/ou l'élément de fermeture (400) est agencé au niveau d'une extrémité inférieure de la cloison de séparation et/ou l'élément de fermeture (400) est fixé de manière amovible et/ou l'élément de fermeture peut être ouvert par pivotement autour d'un axe de pivotement (S).

4. Doseur volumétrique (9) selon au moins l'une quelconque des revendications précédentes,
dans lequel la cloison de séparation réglable en hauteur (301) est réalisée sous forme de premier montant d'un élément de transmission de force.

5. Doseur volumétrique selon la revendication précédente,
dans lequel l'élément de transmission de force (300) présente un deuxième montant (320), qui est relié au premier montant (310).

6. Doseur volumétrique (9) selon au moins l'une quelconque des deux revendications précédentes,
dans lequel un élément de réglage (700) est agencé au niveau de l'élément de transmission de force (300), en particulier au niveau de son deuxième montant (320).

7. Doseur volumétrique (9) selon la revendication précédente,
dans lequel le premier (310) et le deuxième montant (320) de l'élément de transmission de force (300) sont reliés l'un à l'autre par le biais d'un arc de raccordement (330), et/ou
dans lequel l'élément de transmission de force (300) est réalisé de telle sorte qu'un réglage en hauteur du premier montant (310) entraîne un réglage en hauteur opposé correspondant du deuxième montant (320).

8. Doseur volumétrique (9) selon au moins l'une quelconque des deux revendications précédentes,
dans lequel le deuxième montant (320) de l'élément de transmission de force (300) est agencé au niveau d'un côté extérieur du récipient d'aliments pour animaux (900), en particulier au niveau d'un côté extérieur de la deuxième section de récipient (200).

9. Doseur volumétrique (9) selon la revendication 6,
dans lequel un élément de fixation (720) est prévu au niveau de l'élément de réglage pour la fixation du deuxième montant de l'élément de transmission de force (300) et/ou de l'élément de réglage.

10. Doseur volumétrique (9) selon au moins l'une quelconque des revendications précédentes,
dans lequel le récipient d'aliments pour animaux (900) présente une trémie de distribution (960), au niveau de l'extrémité inférieure de laquelle la sortie d'aliments pour animaux (210) est agencée.

11. Doseur volumétrique (9) selon au moins l'une quelconque des revendications précédentes,
dans lequel le doseur volumétrique présente un élément de poids (950) pour la fermeture de la sortie d'aliments pour animaux (210).

12. Installation d'alimentation pour animaux (10) pour la mise à disposition d'aliments pour animaux, en particulier d'aliments secs pour cochons, en particulier pour l'alimentation individuelle des animaux, comprenant une pluralité d'auges (16) et une conduite d'alimentation d'aliments pour animaux (13) pour la mise à disposition d'aliments pour animaux à la pluralité d'auges (16), dans lequel un doseur volumétrique (9) selon au moins l'une quelconque des revendications précédentes est prévu au niveau d'une, de plusieurs ou de toutes les auges (16).

13. Système de dosage volumétrique variable pour une installation d'alimentation pour animaux (10) pour la mise à disposition d'aliments pour animaux, en particulier d'aliments secs pour cochons, en particulier pour l'alimentation individuelle des animaux, le système de dosage volumétrique variable comprenant
un doseur volumétrique (9) comprenant
- un récipient d'aliments pour animaux (900) avec une première section de récipient (100) avec une entrée d'aliments pour animaux (110) et une deuxième section de récipient (200) avec une sortie d'aliments pour animaux (210) pouvant être fermée pour la distribution par gravité d'aliments pour animaux,
- dans lequel la première section de récipient (100) présente une ouverture de récipient (120), laquelle relie la première section de récipient (100) à la deuxième section de récipient (200) pour un passage par gravité d'aliments pour animaux de la première section de récipient (100) à la deuxième section de récipient (200),
- dans lequel une cloison de séparation réglable en hauteur (301) est agencée entre la première (100) et la deuxième section de récipient (200), dans lequel la cloison de séparation réglable en hauteur est orientée sensiblement à la verticale dans un état de fonctionnement du doseur volumétrique,
- dans lequel un réglage en hauteur de la cloison de séparation augmente un volume de la première section de récipient (100) et diminue un volume de la deuxième section de récipient (200), et inversement,
**caractérisé en ce que**
pour la distribution par gravité des aliments pour animaux à partir de la première section de récipient, il faut ouvrir non seulement la sortie d'aliments pour animaux mais aussi l'ouverture de récipient,
le système de dosage volumétrique variable comprenant en outre un module de dosage minimum présentant un élément de fermeture, qui est réalisé pour être agencé au niveau de la cloison de séparation pour fermer l'ouverture de récipient (120).

14. Procédé de mise à disposition d'aliments pour animaux, en particulier d'aliments secs pour cochons, en particulier pour l'alimentation individuelle des animaux,
le procédé comprenant :
- la fermeture d'une sortie d'aliments pour animaux (210) réalisée au niveau d'une deuxième section de récipient (200) d'un récipient d'aliments pour animaux (900) d'un doseur volumétrique (9) pour la distribution par gravité d'aliments pour animaux,
- la fermeture d'une ouverture de récipient (120) pour un passage par gravité d'aliments pour animaux d'une première section de récipient (100) à la deuxième section de récipient (200) par un élément de fermeture (400), qui est agencé au niveau d'une cloison de séparation agencée entre la première (100) et la deuxième section de récipient (200),
- le réglage en hauteur de la cloison de séparation (301), dans lequel la cloison de séparation réglable en hauteur est orientée sensiblement à la verticale dans un état de fonctionnement du doseur volumétrique,
- le transfert d'aliments pour animaux par une entrée d'aliments pour animaux (110) dans la première section de récipient (100),
- l'ouverture de l'ouverture de récipient (120) et de la sortie d'aliments pour animaux (210) pour la distribution par gravité des aliments pour animaux à partir de la première section de récipient.

15. Procédé de variation d'un système de dosage volumétrique, le procédé comprenant :
la mise à disposition d'un doseur volumétrique comprenant
- un récipient d'aliments pour animaux (900) avec une première section de récipient (100) avec une entrée d'aliments pour animaux et une deuxième section de récipient (200) avec une sortie d'aliments pour animaux (210) pouvant être fermée pour la distribution par gravité d'aliments pour animaux,
- dans lequel la première section de récipient (100) présente une ouverture de récipient (120), laquelle relie la première section de récipient (100) à la deuxième section de récipient (200) pour un passage par gravité d'aliments pour animaux de la première section de récipient (100) à la deuxième section de récipient (200),
- dans lequel une cloison de séparation réglable en hauteur (301) est agencée entre la première (100) et la deuxième section de récipient (200), dans lequel la cloison de séparation réglable en hauteur est orientée sensiblement à la verticale dans un état de fonctionnement du doseur volumétrique, et dans lequel pour la distribution par gravité des aliments pour animaux à partir de la première section de récipient, il faut ouvrir non seulement la sortie d'aliments pour animaux mais aussi l'ouverture de récipient,
- dans lequel un réglage en hauteur de la cloison de séparation (301) augmente un volume de la première section de récipient (100) et diminue un volume de la deuxième section de récipient (200), et inversement,
le procédé comprenant en outre
- l'agencement d'un élément de fermeture (400) d'un module de dosage minimum au niveau de la cloison de séparation pour la fermeture de l'ouverture de récipient (120).
